(19) Europäisches Patentamt European Patent Office Office européen des brevets

(11) **EP 3 658 893 B1**

(12) **EUROPEAN PATENT SPECIFICATION**

(45) Date of publication and mention of the grant of the patent:
**10.05.2023 Bulletin 2023/19**

(21) Application number: **18838205.5**

(22) Date of filing: **23.07.2018**

(51) International Patent Classification (IPC):
**G01N 21/3581** *(2014.01)*   **G01N 33/08** *(2006.01)*
**A01K 43/04** *(2006.01)*   **B65G 47/91** *(2006.01)*

(52) Cooperative Patent Classification (CPC):
**G01N 21/3581; A01K 43/04; G01J 3/28;
G01N 33/08; B65G 47/91**

(86) International application number:
**PCT/IL2018/050814**

(87) International publication number:
**WO 2019/021275 (31.01.2019 Gazette 2019/05)**

(54) **A SYSTEM AND METHOD FOR NON-INVASIVELY DETERMINING EGG PROPERTIES**

SYSTEM UND VERFAHREN ZUR NICHT-INVASIVEN BESTIMMUNG DER EIGENSCHAFTEN VON EIERN

SYSTÈME ET PROCÉDÉ DE DÉTERMINATION NON INVASIVE DES PROPRIÉTÉS D'UN OEUF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **23.07.2017 US 201762535917 P**

(43) Date of publication of application:
**03.06.2020 Bulletin 2020/23**

(73) Proprietor: **Terahertz Group Ltd.
Herzliya 4685160 (IL)**

(72) Inventor: **GABBAI, Eran
Herzeliya, 4650447 (IL)**

(74) Representative: **Mewburn Ellis LLP
Aurora Building
Counterslip
Bristol BS1 6BX (GB)**

(56) References cited:
**WO-A1-2015/145435**      **WO-A1-2018/023105**
**DE-A1-102012 023 947**      **US-A1- 2004 107 912**

- **BEN WEBSTER ET AL: "Avian Egg Odour Encodes Information on Embryo Sex, Fertility and Development", PLOS ONE, vol. 10, no. 1, 28 January 2015 (2015-01-28), pages 1-10, XP055380142, DOI: 10.1371/journal.pone.0116345**
- **YOU BORWEN ET AL: "Terahertz volatile gas sensing by using polymer microporous membranes", OPTICS EXPRESS, vol. 23, no. 3, 26 January 2015 (2015-01-26), page 2048, XP055781233, DOI: 10.1364/OE.23.002048**
- **WEBSTER, BEN et al.: "Avian egg odour encodes information on embryo sex, fertility and development", PloS one, vol. 10, no. 1, 28 January 2015 (2015-01-28), page 01 16345, XP055380142,**
- **COSTANZO, ALESSANDRA et al.: "The odour of sex: sex-related differences in volatile compound composition among barn swallow eggs carrying embryos of either sex", PloS one, vol. 11, no. 11, 11 November 2016 (2016-11-11), XP055567136,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 658 893 B1

## Description

## TECHNOLOGICAL FIELD

The present invention relates to a system and method for non-invasively determining egg properties.

## BACKGROUND ART

WO 2018/023105 A1 discloses a system according to the preamble of claim 1. Further references considered to be relevant as background to the presently disclosed subject matter are listed below:

- US patent 4,955,728

- US patent 4,671,652

- US 2016/0050891 A1

- US 2004/0107912 A1

- US 2009/0091742 A1

- PCT application publication WO 2014/086335 A1

- PCT application publication WO 2015/145435 A1

- "Gender Identification of Chicks Prior to Hatch" 50™ Annual National Breeders Roundtable St. Louis, Missouri May 3-4, 2001

- Webster B, Hayes W, Pike TW (2015) "Avian Egg Odour Encodes Information on Embryo Sex, Fertility and Development." PLoS ONE 10(1): e0116345.

- Opt Express. 2015 Feb 9;23(3):2048-57. doi: 10.1364/OE.23.002048 "Terahertz volatile gas sensing by using polymer microporous membranes."

## BACKGROUND

Eggs which are to be hatched to live poultry are typically candled during embryonic development to identify clear, rotten, and dead eggs (collectively referred to herein as "non-live eggs"). Non-live eggs are removed from incubation to increase available incubator space. U.S. Patent No. 4,955,728 to Hebrank, describes a candling apparatus that uses infrared detectors and the infrared radiation emitted from an egg to identify live eggs. U.S. Patent No. 4,671,652 to van Asselt et al. describes a candling apparatus in which a plurality of light sources and corresponding light detectors are mounted in an array, and wherein eggs are passed between the light sources and the light detectors to identify live eggs.

In commercial hatcheries, eggs typically are held in setting flats during incubation. At a selected time, typically on the eighteenth day of incubation, the eggs are removed from an incubator. Unfit eggs (namely, dead eggs, rotten eggs, empty eggs, and clear eggs) are identified and removed, and live eggs are treated (e.g., inoculated) and then transferred to hatching baskets.

In hatchery management, it may be desirable to separate birds based upon various characteristics, such as gender, diseases, genetic traits, etc. For example, it may be desirable to inoculate male birds with a particular vaccine and inoculate female birds with a different vaccine. Sex separation of birds at hatch may be important for other reasons as well. For example, turkeys are conventionally segregated by sex because of the difference in growth rate and nutritional requirements of male and female turkeys. In the layer or table egg industry, it is desirable to keep only females. In the broiler industry, it is desirable to segregate birds based on sex to gain feed efficiencies, improve processing uniformity, and reduce production costs.

Unfortunately, conventional methods of sexing birds may be costly, labor intensive, time consuming, and typically require trained personnel with specialized skills. Conventional methods of sexing birds include feather sexing, vent sexing, and DNA or blood sexing. About three-thousand (3,000) chicks can be feather-sexed per hour at a cost of about 0.7 to 2.5 cents per chick. About fifteen hundred (1,500) chicks can be vent-sexed per hour at a cost of about 3.6 to 4.8 cents per chick. DNA or blood sexing is performed by analyzing a small sample of blood collected from a bird.

It would be desirable to identify the sex of birds, as well as other characteristics of birds, prior to hatching. Pre-hatch sex identification could reduce costs significantly for various members of the poultry industry, and reduce or even eliminate unwanted male chicks kill. Although conventional candling techniques can discriminate somewhat effectively between live and non-live eggs, these conventional candling techniques may not be able to reliably determine gender and other characteristics of unhatched birds.

## GENERAL DESCRIPTION

The present invention is defined in claims 1 and 7 and relates to the use of Terahertz (THz) radiation in avian gender classification. The term "THz radiation" generally refers herein below to any of the electromagnetic wave frequencies that lie in the range extending from around 100 GHz to 30 THz. More specifically, there is provided a method and a system for collecting a volatile organic compound and/or mix of compounds and applying THz based detection of a signature of the collected compounds to determine egg properties. Egg properties include gender and/or fertility. The term "volatiles" or "VOCs" generally refers herein below to volatile organic compound and/or mix of compounds.

Volatiles emitted from developing eggs convey information on egg fertility, along with the sex and developmental status of the embryo. Specifically, egg volatiles which changed over the course of incubation, differed between fertile and infertile eggs, and were predictive of embryo sex as early as immediately after laying at day 1 and prior to incubation.

The technique of the present invention is capable of detecting volatiles prior to egg incubation due to the THz spectroscopy technique being capable of detection of materials/compounds at very low concentrations, below PPB (parts per billion).

THz technology enables to differentiate between eggs containing male and female embryos along with non-fertile eggs on day 1. Volatile organic compounds (VOCs) originating and emitted from the eggs, carry information regarding fertility and gender. These VOCs are collected through the porous egg shell. Each separate gender, and also the non-fertile eggs, has a unique mixture of VOCs which may be identified with THz technology. In general, the eggs are sampled using vacuum suction, and the VOCs are trapped in a pressure dischargeable capacitor. The collection system comprises a vacuum gripper carrying a pressure dischargeable capacitor being configured as a pressure permeable membrane, such that when the egg is held by the vacuum gripper (i.e. held by suction) the pressure dischargeable capacitor is located at the propagation path of the VOCs released from the egg through the eggshell. The vacuum accelerates the flow of the volatiles and the use of the pressure dischargeable capacitor provides for trapping the collected volatile organic compounds within the membrane upon releasing the negative pressure (i.e. vacuum). Then the eggs are scanned with THz waves and the specific VOCs for each gender and/or non-fertile eggs are detected based on the individual fingerprints adsorption. The pressure dischargeable capacitor is then capable of releasing/discharging the trapped vapors by an operation including also positive or negative pressure. It should be noted that without application of pressure (positive or negative), the pressure dischargeable capacitor is not capable of trapping or releasing any material.

Therefore, according to the present invention, there is provided a system for determining one or more egg properties prior to incubation. The system comprises at least one vacuum gripper carrying a pressure dischargeable capacitor and a control unit configured and operable for receiving data indicative of the collected volatile organic compounds being scanned with an electromagnetic radiation in the THz range, and processing the data for identifying a signature being indicative of at least one egg property to thereby generate information data being indicative of at least one egg property. The vacuum gripper is configured and operable to hold an egg by suction and the pressure dischargeable capacitor is located at the propagation path of volatile organic compounds released by the egg. The pressure dischargeable capacitor is configured and operable for trapping the collected volatile organic compounds.

The term *"pressure dischargeable capacitor"* refers to a pressure permeable membrane being capable of trapping volatile compounds therein upon releasing a negative pressure and of releasing/discharging such trapped volatile compounds, when desired, upon application of pressure, including positive pressure.

In some embodiments, the control unit is configured and operable for performing a pattern recognition of the signature.

In some embodiments, the pressure dischargeable capacitor is configured and operable for trapping the collected volatile organic compounds within a period of time being less than a period of time spent for transporting the egg from a tray to a conveyor. The period of time may be less than 5 seconds.

In some embodiments, the system further comprises a spectroscopic assembly including a radiation transmitter unit being configured and operable to scan the permeable capacitor holding the collected volatile organic compounds by generating an electromagnetic radiation in the range of THz within a scanning window of about 100 GHz and a detection unit being configured and operable to detect an electromagnetic radiation emitted by the collected volatile organic compounds.

According to the present invention the detection unit is located at a distance from the permeable capacitor, which is less than the wavelength of the THz radiation.

In some embodiments, the pressure dischargeable capacitor has a thickness being at least several times the wavelength of the electromagnetic radiation.

According to the present invention, there is provided a method for determining one or more egg properties prior to incubation. The method comprises receiving data indicative of collected volatile organic compounds being scanned with electromagnetic radiation in the THz range and processing the data for identifying a signature being indicative of at least one of gender and fertility.

In some embodiments, the step of processing the data for identifying a signature comprises performing pattern recognition of the signature.

In some embodiments, the method further comprises performing a THz spectroscopy of the egg.

In some embodiments, the method further comprises scanning the collected volatile organic compounds with electromagnetic radiation in the THz range within a scanning window of about 100 GHz.

In some embodiments, the method further comprises trapping collected volatile organic compounds by suction, wherein the trapping is performed within a period of time being less than a period of time spent for transporting the egg from a tray to a conveyor.

## BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:

Fig. 1 exemplifies a block diagram of a system of an embodiment of the present invention for determining egg properties prior to incubation;

Fig. 2A is an image exemplifying the vacuum gripper of an embodiment of the present invention;

Fig. 2B is an image exemplifying the pressure dischargeable capacitor of the present invention;

Fig. 3A illustrates a flow chart exemplifying a technique for determining egg properties prior to incubation;

Fig. 3B illustrates a flow chart exemplifying a technique for pattern recognition according to some embodiments of the present invention;

Figs. 4A-4C show THz spectra obtained from an egg for a male, female and a reference capacitor respectively;

Figs. 5A-5B show female and male THz signatures respectively obtained by using the teachings of the present invention; and

Fig. 6 shows a graph illustrating pattern classification stage and gender differentiation obtained by using the teachings of the present invention.

## DETAILED DESCRIPTION OF EMBODIMENTS

Because the illustrated embodiments of the present invention may, for the most part, be implemented using electronic components and circuits known to those skilled in the art, details will not be explained in any greater extent than that considered necessary as illustrated above, for the understanding and appreciation of the underlying concepts of the present invention and in order not to obfuscate or distract from the teachings of the present invention.

Any reference in the specification to a method should be applied mutatis mutandis to a system capable of executing the method.

Any reference in the specification to a system should be applied mutatis mutandis to a method that may be executed by the system.

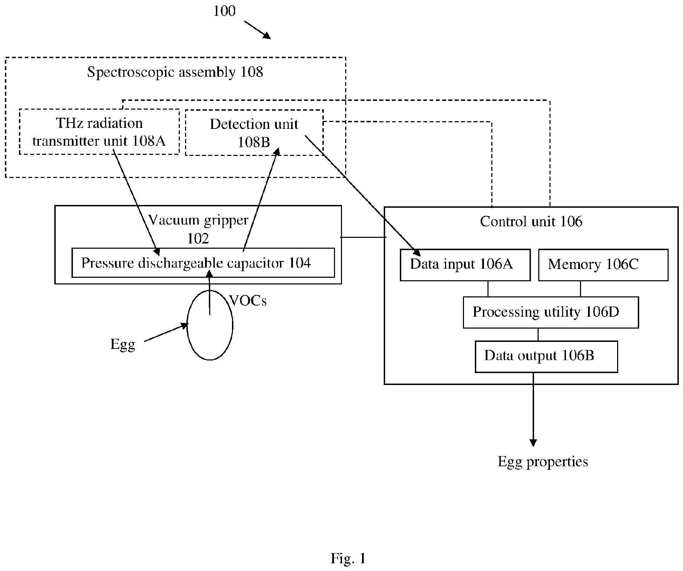

Referring to Fig. 1, there is illustrated, by way of a block diagram, a system 100 of an embodiment of the present invention configured and operable for determining egg properties prior to incubation in ovo. The system 100 includes a vacuum gripper 102 carrying a pressure dischargeable capacitor 104 and a control unit 106 configured and operable for receiving data indicative of volatile organic compounds released by the egg being scanned with an electromagnetic radiation in the THz range and processing the data for identifying a THz signature being indicative of at least one egg property to thereby generate information data being indicative of at least one egg property. Terahertz (THz) radiation is known to interact with polar molecules via rotational or/and vibrational transition levels. These interactions are manifested as absorption. The frequency THz spectrum obtained by scanning the pressure dischargeable capacitor is indicative of various chemical materials including volatile organic compounds having individual specific fingerprints.

As will be described more specifically further below, the control unit 106 is configured to receive and process the response signal emitted by the egg and identify spectral special features indicative of a THz signature of the egg. For example, the THz signature may include information on the gender and/or the fertility of the egg. The information included in the THz signature is thus associated with the sorting process. The system 100 is configured to be used with at least one egg having properties identifiable by THz inspection, such that upon examination by THz analysis, the gender and/or the fertility of the egg may be identified. The inventors found that each gender (male or female) and/or the fertility of egg has its own THz signature.

In some embodiments, the control unit 106 is configured and operable for performing a pattern recognition of the THz signature. The control unit 106 is configured generally as a computing/electronic utility including inter alia such utilities as data input and output utilities 106A, 106B, memory 106C, and data processing utility 106D. The utilities of the control unit 106 may thus be implemented by suitable circuitry and/or by software and/or hardware components including computer readable code configured for implementing the operations of methods 200 and/or 300 described below.

The features of the present invention may comprise a general-purpose or special-purpose computer system including various computer hardware components, which are discussed in greater detail below, and which may also include computer-readable media for carrying or having computer-executable instructions, computer-readable instructions, or data structures stored thereon. Such computer-readable media may be any available media, which are accessible by a general-purpose or special-purpose computer system. By way of example, without limitation, such computer-readable media can comprise physical storage media such as RAM, ROM, EPROM, flash disk, CD-ROM or other optical disk storage, magnetic disk storage or other magnetic storage devices, or any other media which can be used to carry or store desired program code means in the form of computer-executable instructions, computer-readable instructions, or data structures and which may be accessed by a general-purpose or special-purpose computer system. Computer-readable media may include a computer program or computer application downloadable to the computer system over a network, such as a wide area network (WAN), e.g. Internet.

In this description and in the following claims, a "control unit" is defined as one or more software modules, one or more hardware modules, or combinations thereof, which work together to perform operations on electronic data. For example, the definition of processing utility includes the hardware components of a personal computer, as well as software modules, such as the operating system of a personal computer. The physical layout of the modules is not relevant. A computer system may include one or more computers coupled via a computer network. Likewise, a computer system may include a single physical device where internal modules (such as a memory and processor) work together to perform operations on electronic data. While any computer system may be mobile, the term "mobile computer system" or the term "mobile computer device" as used herein especially includes laptop computers, netbook computers, cellular telephones, smartphones, wireless telephones, personal digital assistants, portable computers with touch sensitive screens, and the like.

The control unit **106** of the present invention may be implemented as part of a signal processing center, and/or as a portable (e.g. handheld) THz reading device. Data input utility **106A** includes a communication module for receiving the response THz signal, an optional data output utility **106B** for generating data relating to identified egg(s), a memory (i.e. non-volatile computer readable medium) **106C** for storing a learning database i.e. preselected data indicative of THz signatures of the eggs versus the eggs properties, and a data processing utility **106D** adapted for identifying the gender and the fertility of the egg(s). The database may be implemented with Microsoft® Access®, Cybase®, Oracle® or other suitable commercial database systems. In some embodiments the system **100** is configured in a cloud-based configuration and/or utilize Internet based computing so that parts of processing utility **106D,** and/or memory **106C** may reside in multiple distinct geographic locations. After the THz response signal(s) is/are received, the data processing utility **106D** is enabled to process the signal(s). Results of the signal processing step may be displayed and/or stored in storage and/or sent to a data communication unit for transfer to a sorting device. The memory **106C** may include instructions executable by data processing utility **106D.** The instructions may be operable to enable data processing utility **106D** to receive the THz response signal(s), to process the THz response signal (s), to identify at least one egg property, and to output via the data output utility **106B** a notification regarding the property of the egg. The notification may be directly sent to a sorting device for sorting the eggs accordingly. Memory **106C** and may be relayed via wireless or wired connection by an external unit to a central database.

In some embodiments, the control unit **106** activates a spectroscopy assembly **108** configured and operable for obtaining the THz signature. Spectroscopic assembly **108** may or may not be a part of the system of the present invention. The processing utility **106D** signals to THz radiation transmitter unit **108A** to emit THz radiation passing though the pressure dischargeable capacitor **104** (being in the optical path of the THZ radiation). Data input **106A** receives a radiation signal pattern via radiation detection unit **108B.** The radiation signal pattern is the radiation that was not adsorbed by the pressure dischargeable capacitor **104.** The radiation signal pattern contains the THz signature. Processing utility **106D** may transmit data regarding the signal pattern (such as gender and/or fertility) via the data output utility **106B,** via a data communication (e.g. via cellular network) to a communication module of a central computer. The processing utility **106D** may record the received data in a learning database in memory **106C** and/or may query/cross-reference the received data with data in the learning database to identify the egg properties and may communicate such egg data to a mobile device at which processing utility **106D** may signal to display a message corresponding to the egg data. To this end, the preselected data stored in the learning database may be used to compare the THz pattern/ signature of the collected volatile organic compounds with the signatures of egg properties stored in the learning database.

Vacuum gripper **102** is configured and operable to hold an egg by suction. The pressure dischargeable capacitor **104** is configured as a permeable capacitor being capable of trapping the collected volatile organic compounds. The fertilized eggs are thus subjected to negative pressure at the external surface of the egg to create a vacuum. A pressure cup, such as a suction cup, may contain the vacuum gripper and can be aligned above the external shell surface, preferably immediately over the air sac. The vacuum gripper is then displaced downwards towards the egg and a negative pressure vacuum is then applied through the pressure dischargeable capacitor, trapping volatiles from the air sac and other portions of the egg. The working range of vacuums which may be employed is about 600 mmHg for a short time period. The time range for application of these vacuums is from about one second to five seconds.

The pressure dischargeable capacitor **104** is a pressure permeable membrane and may be configured as a dense, compressed structure made of fibers (e.g. mesh) such that the pressure permeable membrane responds to the application/release of vacuum as a pressure dischargeable capacitor. The pressure dischargeable capacitor may be configured as a metamaterial membrane being a material deriving its properties not from the properties of the basic materials, but from its designed structure. The metamaterial membrane may comprise a plurality of layers of metamaterial encapsulated in a plastic housing, produced with an accuracy of $\pm$ 10 microns.

In some embodiments, the system **100** includes a spectroscopic assembly **108** including a radiation transmitter unit **108A** being configured and operable to produce THz frequency radiation and a detection unit **108B** being configured and operable to detect an electromagnetic radiation emitted by the collected volatile organic compounds. In particular, radiation transmitter unit **108A** is operable for irradiating the egg with a radiation having a wavelength in the range extending from around 100 GHz to 30 THz and to scan the permeable capacitor holding the collected volatile organic compounds within a scanning window of about 100 GHz. Although, for the sake of clarification, the radiation transmitter unit **108A** and the detection unit **108B** are represented as two separate physical elements, they can be integrated in the same physical element or in the same housing. In a specific and non-limiting example, radiation transmitter unit **108A** is configured and operable for generating inspecting and reference electro-magnetic radiation components of substantially the same frequency contents, and for sweeping/scanning the frequency. Detection unit **108B** may be located in a first path of the inspecting radiation components after passing through the pressure dischargeable capacitor **104** and in a second path of the reference radiation component directly propagating from the transmitter unit **108A**. The spectroscopic assembly **108** may be configured to induce a predetermined frequency difference between a frequency of the inspecting radiation component and the reference radiation component interacting at the detection unit **108B** such that a signal resulting from the interaction between the inspecting and reference components is indicative of one or more properties of the egg at a location where the inspecting radiation interacts with the egg. For example, the spectroscopic assembly **108** may be implemented by the spectroscopic assembly described in US patent No: 9,279,723 B2.

The system **100** of the present invention may comprise the spectroscopy assembly **108** as described above or may directly receive data emitted by the collected volatile organic compounds obtained by an external spectroscopy assembly as described above or as conventionally used in the field. For example, one spectroscopy method is to radiate THz waves directly on the eggshell itself and acquire their spectral information, such as the fingerprint feature or decay signals of a pulse as response signals. The spectroscopy systems include photo-mixing, heterodyne detection, and chirped-pulse THz spectroscopy. Another spectroscopy method is to use the THz resonance field in a photonic crystal, a waveguide device or frequency multiplier.

The pressure dischargeable capacitor **104** is located at the propagation path of the volatile organic compounds. The pressure dischargeable capacitor **104** is also positioned within the optical path of the electromagnetic radiation emitted by the transmitter unit **108A.**

For example, the pressure dischargeable capacitor **104** may be spaced-apart from spectroscopic assembly **108.** The pressure dischargeable capacitor **104** is interrogated by the spectroscopic assembly **108** at some location which can be distant from the vacuum gripper and the response signal carrying the THz signature is transmitted to the control unit **106** via wired/wireless connection or via a communication network. Alternatively, the pressure dischargeable capacitor **104** may be a part of the spectroscopic assembly **108.** In this case, spectroscopic assembly **108** comprises a sample holder on which the pressure dischargeable capacitor **104** is located and examined.

In some embodiments, the system is connectable to a communication network with a host computer, which is external to the control unit **106.** Alternatively, the spectroscopic assembly **108** can be also attached to the control unit **106** by using a coupling member of any type. The control unit **106** is configured and operable to control the operation of the spectroscopic assembly **108** and optionally also of the vacuum gripper **102.** The control unit **106** may be integrated within the spectroscopic assembly **108** or may be a separate element communicating with the spectroscopic assembly **108** via wired or wireless communication. If the control unit **106** is integrated within the spectroscopic assembly **108,** THz signature identification does not require or employ any type of electronic components, circuitry or antenna. It is not shown in detail, but should be appreciated, that signal exchange and communication is enabled between the modules of the system by virtue of appropriate wiring, or wirelessly. For example, the spectroscopic assembly **108** and the control unit **106** can be connected by IR (Infra-Red), RF (radio frequency including Bluetooth®) or cable control. If the spectroscopic assembly **108** and the control unit **106** are integrated in the same physical housing, the THz signature is stored in the control unit **106.** The connections as discussed herein may be any type of connection suitable to transfer signals from or to the respective nodes, units or devices, for example via intermediate devices. Accordingly, unless implied or stated otherwise, the connections may for example be direct connections or indirect connections. The connections may be illustrated or described in reference to being a single connection, a plurality of connections, unidirectional connections, or bidirectional connections. However, different embodiments may vary the implementation of the connections. For example, separate unidirectional connections may be used rather than bidirectional connections, and vice versa. Also, a plurality of connections may be replaced with a single connection that transfers multiple signals serially or in a time multiplexed manner. Likewise, single connections carrying multiple signals may be separated out into various different connections carrying subsets of these signals. Therefore, many options exist for transferring signals.

The transmitter unit **108A** is placed at a certain distance above the permeable capacitor **104.** The distance between the transmitter unit **108A** and the permeable capacitor **104** may be selected to be at a close proximity being less than the wavelength of the electromagnetic radiation. For example, this distance may be selected to be below 1 mm for a radiation in the range of about 400 GHz to 500 GHz. In a specific and non-limiting example, the distance between the transmitter unit **108A** and the permeable capacitor **104** is selected to be in the range of about 0.599 - 0.749 mm. In this connection, it should be understood that, due to the propagation path in the THz range, if the distance between the transmitter unit **108A** and the permeable capacitor **104** is selected to be less than the wavelength of the electromagnetic radiation, the result signal(s) will be screened from the environment (i.e. not affected by surrounding changes such as changes in humidity, temperature ...), eliminating the need to perform the acquisition of the response signal(s) in a controlled environment (e.g. a clean room such as a hood, or under inert conditions including cleaning with nitrogen or helium gas). Moreover, the short distance between the transmitter unit **108A** and the permeable capacitor **104** eliminates the absorbance of the THz signal by the environment.

Moreover, in some embodiments, the thickness of the pressure dischargeable capacitor **104** may be selected to be at least several times (e.g. at least four times) the wavelength of the electromagnetic radiation. The thickness should be selected to be sufficiently wide to enable to capture a sufficient amount of volatile organic compounds allowing to perform an analysis providing an identifiable THz signature. For example, the thickness of the pressure dischargeable capacitor **104** may be selected to be 3-4 mm for a radiation in the range of about 400 GHz to 500 GHz.

In some embodiments, the system **100** comprises a plurality of vacuum grippers **102** carried by a tray being positioned on top of a plurality of eggs supported by a conveyor. The vacuum grippers **102** and the eggs are aligned such that at each inspection cycle, the tray carrying a plurality of vacuum grippers (e.g. arranged linearly or in a matrix manner) moves down towards the eggs such that each vacuum gripper holds, by suction, one egg at a time. In this case, the control unit **106** will receive together with the THz signature the position of the egg on the conveyor to enable an adequate sorting thereafter.

In some embodiments, the spectroscopy assembly **108** and the vacuum gripper **102** are located in the same inspection chamber, such that analysis of the volatile organic compounds is performed in real-time. Alternatively, the pressure dischargeable capacitor **104** may be released from the vacuum gripper **102** and inspected in a separate inspection chamber after trapping of the volatile organic compounds.

In some embodiments, the permeable capacitor **104** is configured and operable for trapping the collected volatile organic compounds within a period of time being less than a period of time spent for transporting the egg from a tray to a conveyor. In this connection, it should be noted that the capability of the system to identify a THz signature, provides a fast inspection rate, being a significant parameter for commercial use in the poultry industry. It should be understood that, as described above, the THz radiation is capable of providing an identifiable signature even when the collected volatile organic compounds are present in the vapor collection in a very-low concentration below PPB. In other words, the THz signature is sensitive to low changes in the vapor composition and provides a detection with high resolution. The high resolution of the THz signature enables to differentiate between signatures of different genders. If the resolution of the signature is not good enough, the THz signatures would overlap and a differentiation between them is then impossible. By contrast, the use of infrared radiation does not provide an identifiable signal. A spectroscopic analysis using an infrared radiation including the collection of the gas and the separation of the different chemical components, yields poor results. Moreover, the high rate of gas delivery required by the infrared spectroscopy does not permit collection of the carrier and separated components in a small area. Furthermore, the period of time for collecting a certain amount of volatile organic compounds which can be spectroscopically analyzed by using infrared radiation, is much higher. For example, the time consumed to be able to obtain an identifiable infrared spectral data is about half an hour. In addition, the concentration of the volatile organic compounds in the aforementioned approach is too low to yield adequate infrared absorption. In other words, much higher concentrations are needed to provide an identifiable signal. The use of Raman techniques can provide an identifiable signal even with low concentrations of the volatile organic compounds, however, the data collection time is much longer than with the technique of the present invention and is therefore not suitable for commercial use in which the rate of egg sorting is an important parameter. Moreover, it should be noted that techniques known in the art using THz spectroscopy provide a spectral analysis of each chemical component of the collected volatile organic compounds, separately indicating the presence of concentration of each collected volatile organic compound, which is highly time consuming. Since the period of time spent for trapping a minimal quantity of collected volatile organic compounds being in a sufficient concentration for providing an identifiable signature is less than a period of time spent for transporting the egg from a tray to a conveyor, the technique of the present invention does not increase the total time of the typical transport process. For example, if the vacuum handling for transport of eggs before being sorted and conveyed to the incubator is less than 5 seconds (e.g. 3 second), the period of time spent for trapping a minimal quantity of collected volatile organic compounds is also less than 5 seconds (e.g. 3 second) and can be integrated within a sorting process of eggs before incubation.

The control unit **106** may comprise a sorter which controls a diverting apparatus located at the end of an outlet conveyor. The sorter accesses data generated by the processing utility **106D** indicative of one or more properties of the egg in the learning database stored in a memory and uses the data to execute the sorting. The data may also include sorting parameters, and the results of comparison. Once the data is stored in the learning database, such data may be analyzed using known database analysis tools, such as a query language, such as, for example, Microsoft® SQL®.

The sorter determines according to this comparison, which eggs are female or male, and, if the eggs are female, their fertility. The sorter is thus configured to receive from the processing utility **106D** via a communication module, data indicative of the one or more egg properties, and controls operation of the diverting apparatus to selectively divert the sorted eggs. The sorter may then classify the eggs, and activate the diverting apparatus. The diverting apparatus diverts the eggs from a continuous track towards a different track or collection means. The diverter apparatus may take the form of a shaft of a solenoid that is notched so that it either forms a continuation of the track, or a barrier on the track. The functions of the sorter may be performed by processing utility **106D** forming a single unit.

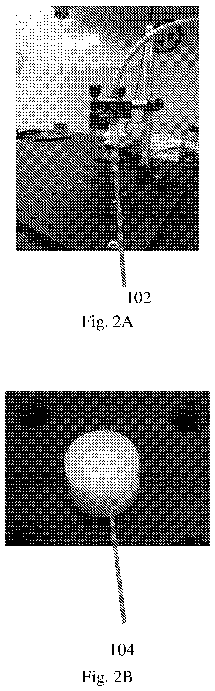

Reference is made to **Fig. 2A** showing a picture exemplifying a vacuum gripper **102** carrying a pressure dischargeable capacitor and being configured for holding an egg by suction. **Fig. 2B** shows a picture exemplifying a pressure dischargeable capacitor **104** being configured as a pressure permeable membrane located at the propagation path of the VOCs released from the egg through the eggshell. As described above, pressure dischargeable capacitor **104** is configured for trapping the collected volatile organic compounds within the membrane upon releasing the negative pressure.

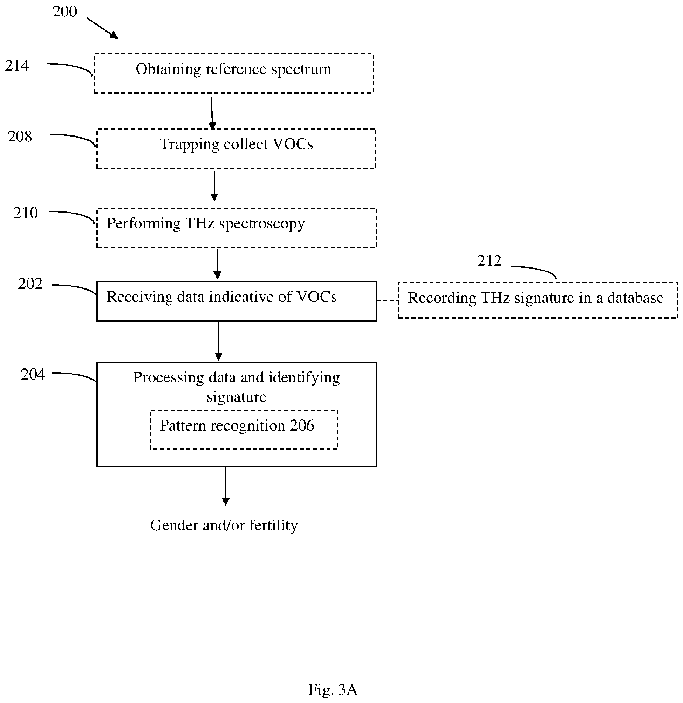

Reference is made to **Fig. 3A** illustrating a flow chart **200** exemplifying a method carried out by the above-described system **100** utilizing the control unit **106** of the invention for identifying a THz spectral signature and determining one or more properties of the egg prior to incubation. This flow chart exemplifies the system operation for generating egg properties data. The method **200** comprises the steps of receiving data indicative of collected volatile organic compounds being scanned with electromagnetic radiation in the THz range in step **202** and processing the data for identifying a signature being indicative of at least one of gender and fertility in step **204**. The step **204** of processing may comprise the step **206** of performing a pattern recognition of the signature.

In some embodiments, prior to step **202** of receiving data indicative of collected volatile organic compounds, the method **200** further comprises performing a THz spectroscopy of the egg in step **210**. This may be implemented by scanning the collected volatile organic compounds captured in the pressure dischargeable capacitor with an electromagnetic radiation in the THz range within a scanning window of about 100 GHz (e.g. by collecting 500 measurements). This narrow scanning window enables to perform a fast scanning of the egg and to reduce the period of time required for performing the inspection process. Moreover, this narrow scanning window also enables fast noise cancellation and an increase in accuracy of the measurements.

In some embodiments, prior to step **210** of performing a THz spectroscopy of the egg, the method **200** may comprise the step **208** of trapping collected volatile organic compounds by suction, wherein the trapping is performed within a period of time being less than a period of time spent for transporting the egg from a tray to a conveyor, as described above.

In some embodiments, prior to step **208** of trapping the collected volatile organic compounds by suction, the method **200** may comprise the step **214** of obtaining a reference spectrum by performing a THz spectroscopy on a reference clean dischargeable/permeable capacitor being the same dischargeable/permeable capacitor used in step **210**. In some embodiments, the method **200** may comprise the step of cleaning a dischargeable/permeable capacitor having trapped volatile compounds for a further use by applying a positive/negative pressure.

In a specific and non-limiting example, performing a THz spectroscopy is implemented by scanning a capacitor and collecting 500 measurements. In step **204**, in which the spectral data is processed, the spectrum of the egg obtained by permeable capacitor filled egg VOCs in step **210** is compared to the reference spectral data obtained in step **214**.

In some embodiments, method **200** may further comprise the step **212** of recording a THz signature in the learning database. The learning database may be configured to provide a THz fingerprint/signature associated with the one or more egg properties. For example, method **200** may include storing in the learning database preselected data indicative of the signature of the signal and/or properties of the egg with the signature. The step **204** of processing the data may further include comparing the received THz data to data in the learning database. Received THz data may be logged in a learning database. Logged received THz data may be used for future analyses of future eggs. Optionally, step **204** of processing the data may further include assessing one or more properties of an egg based on the learning database data. Assessing one or more properties may be performed using a statistical analysis in which received THz data is compared to learning database THz data and a statistical comparison is performed. If a predetermined level of similarity is shown, the THz data is considered to have a certain property. After the step of **210** of performing THz spectroscopy, the capacitor may be discharged of VOCs content via various methods which include desorption of VOCs and discharge with vacuum or high pressure flow.

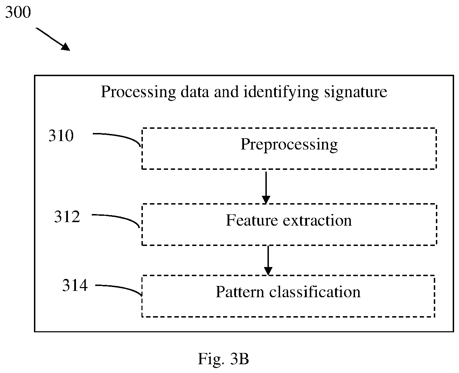

Reference is made to **Fig. 3B** illustrating a flow chart **300** exemplifying a method carried out by the above-described system **100** utilizing the control unit **106** of the invention for identifying a THz spectral signature and determining one or more properties of the egg prior to incubation based on pattern recognition. More specifically, the processing of the control unit **106** comprises the step of providing a mathematical interpretation of pattern recognition based on a learning algorithm such as a Neural Network Acceleration algorithm (NNA). The interpretation of the pattern recognition is based on identification of special features of the pattern such as the identification of main and side peaks, the number of main and side peaks, the width of the peaks and the distance between them.

In some embodiments, the processing step **204** of method **200** above, may comprise the following steps: an optional preprocessing step **310** being configured to remove irrelevant spectral trends present in the measurements, and to filter out random measurement noise; a feature extraction step **312** being configured to estimate the most relevant vectors defining the data using a principal component analysis; and a pattern classification step **314** using a combined linear and nonlinear pattern recognition approach.

In a specific and non-limiting example, the optional preprocessing step **310** may include the step of establishing the learning database. The step of establishing the learning database may comprise the steps of collecting the scans, preprocessing the scans as described above, and performing a Fourier Transformation on the results. The preprocessing step **310** is performed on reference spectral data, obtained in step **214** above and on egg spectral data, obtained in step **210** above. The feature extraction step **312** may include the step of subtracting a reference processed data from the sample processed data. The resulting data belongs to or represents only egg related information (without data relating to the pressure dischargeable capacitor). The step of subtracting the reference processed data (e.g. pressure dischargeable capacitor results) from the sample processed data (e.g. egg sample results) may be followed by a step of performing a second Fourier transformation on the egg related information to provide the specific egg related signals, among them the sex partitioning signals.

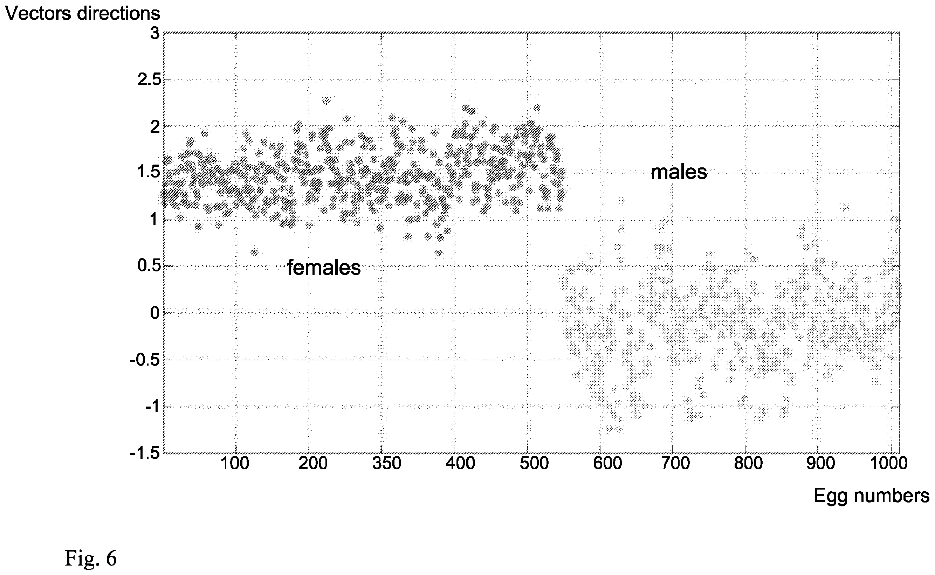

The pattern classification step **314** may include the steps comparing all the obtained results to the learning database. When the learning database is established, the same sampled eggs are tested biologically by Polymerase Chain Reaction (PCR) method for sex determination. Then all the vectors obtained by the mathematical process and the variations (i.e. the mathematically calculated differences) between the samples are "translated" to sex determination and differentiation into two groups as illustrated in **Fig. 6.**

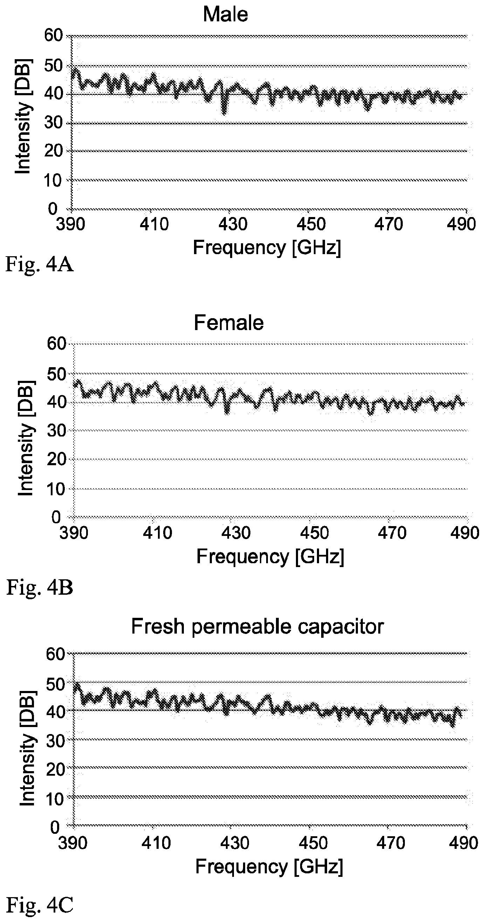

After establishing the learning data base, all next measured eggs data is compared to the learning database by using the same mathematical process. Reference is made to **Figs. 4A-4C** showing THz spectra obtained from an egg for a male **(4A),** female **(4B)** and a non-used capacitor using a reference **(4C)** respectively. The scan process is done on a window of 100GHz, at a range of 390 GHz to 490 GHz, with 0.2GHz step size obtaining a total of 500 points. In particular, the graph of **Fig. 4C** shows an egg's spectrum obtained together with an adjoined calibration spectrum to be used as a control reference.

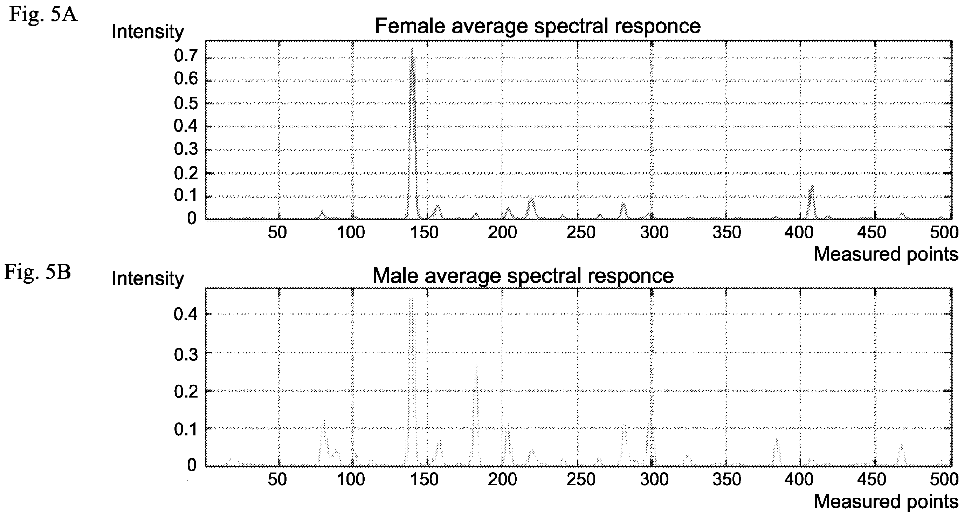

Reference is made to **Figs. 5A-5B** showing a THz signature obtained from an egg for a female **(SA),** male **(SB)** respectively, by using the processing identification technique described with respect to **Fig. 3B** above.

More specifically, **Fig. 5A** shows an average outcome of step **204** as described above after removal of the irrelevant spectral trends present in the measurements and filtering out of the random measurement noise. The X axis represents the 500 measurement points and the Y axis represents the intensity of the Fourier transformation. The calculation for the spikes (peaks) frequency in the above graphs is as follows:

$$\text{Frequency} = 400\text{GHz} + 100\text{GHz}/500\text{points}.$$

As clearly shown in **Figs. 5A-5B,** the technique of the present invention is capable of obtaining different THz signatures being indicative of different properties of an egg. For example, it is shown that the THz signature for a female comprises one main peak after around 140 measurement points. The THz signature for a male comprises a plurality of peaks (about 5) defining a complex THz signature at least after around 140 and 180 measurement points. For example, the pattern classification step may include identifying the gender of the egg by identifying the number of main peaks. In a specific and non-limiting example, when three peaks are identified, the gender of the egg is related to male. When two peaks are identified, the gender of the egg is related to female. The inventors have found that the pattern signal defines a THz signature in which the proportional distance between the main peaks is constant for each gender. More specifically, the difference between male and female and non-fertile eggs results from differences in the ratio of the content of the VOCs. In general, fertile eggs contain more (higher concentration) of alcohols and aromatic compounds versus the non-fertile eggs, which contain same compounds but in much lower concentrations. Female and male eggs contain same VOCs but again differ in the ratio between them. Female eggs contain higher concentrations of long chain ketones and aromatic alcohols and aldehydes. Each mix or blend of VOCs has a separate THz signature which can be translated to distinct peaks of the Fourier transformation. Therefore, the identification of the special features of the pattern such as the number of peaks, the distance between the main peaks, the identification of main and side peaks, the width of the peaks enables to define the properties of the egg. In other words, the inventors have found that obtaining a ratio between the THz signatures of different egg properties enables identification of these properties, and that the specific identification of each VOC component as well as each concentration is not necessary to identify eggs properties. This capability of the system of the present invention is remarkable in the poultry industry since it significantly reduces the time of identification of egg properties.

Reference is made to **Fig. 6** showing a pattern classification stage and gender differentiation obtained by using the pattern classification step **314** of method **300** above. As clearly shown in the figure, the technique of the present invention is capable of differentiating between genders. In particular, the X axis represents the egg number and the Y axis the distance between male and female vectors extracted from step **312** of method **300** above.

In the claims, the word 'comprising' does not exclude the presence of other elements or steps then those listed in a claim. Furthermore, the terms "a" or "an," as used herein, are defined as one or more than one. Also, the use of introductory phrases such as "at least one" and "one or more" in the claims should not be construed to imply that the introduction of another claim element by the indefinite articles "a" or "an" limits any particular claim containing such an introduced claim element to inventions containing only one such element, even when the same claim includes the introductory phrases "one or more" or "at least one" and indefinite articles such as "a" or "an." The same holds true for the use of definite articles. Unless stated otherwise, terms such as "first" and "second" are used to arbitrarily distinguish between the elements such terms describe. Thus, these terms are not necessarily intended to indicate temporal or other prioritization of such elements. The mere fact that certain measures are recited in mutually different claims does not indicate that a combination of these measures cannot be used to advantage.

**Claims**

1. A system (100) for determining one or more egg properties immediately after laying and prior to incubation, the system (100) comprising:

   at least one vacuum gripper (102) carrying a pressure permeable membrane (104), wherein said vacuum gripper (102) is configured and operable to hold an egg by suction and wherein said pressure permeable membrane (104) is located at the propagation path of volatile organic compounds released by the egg, said pressure permeable membrane (104) being configured and operable for trapping the collected volatile organic compounds;
   a THz radiation transmitter (108A) arranged at a first distance from the pressure permeable membrane (104);
   a detection unit (108B) located a second distance from the pressure permeable membrane (104), the detection unit being configured to detect the THz radiation after having passed through the collected volatile organic compounds trapped in the pressure permeable membrane (104); and
   a control unit (106) configured and operable for receiving via the detection unit (108B) a response THz signal indicative of the collected volatile organic compounds being scanned with an electromagnetic radiation in the THz range and processing said response THz signal for identifying a signature being indicative of at least one egg property to thereby generate information data being indicative of at least one egg property;
   **characterized in that** the second distance is less than a wavelength of the THz radiation.

2. The system of claim 1, wherein said control unit (106) is configured and operable for performing a pattern recognition of said signature.

3. The system of claim 1 or claim 2, wherein said pressure permeable membrane (104) is configured to operate for a period of time to trap the collected volatile organic compounds, wherein the period of time is less than a period of time spent for transporting the egg from a tray to a conveyor.

4. The system of claim 3, wherein said period of time is less than 5 seconds.

5. The system of any one of the preceding claims, further comprising a spectroscopic assembly (108) including a radiation transmitter unit being configured and operable to scan said pressure permeable membrane (104) holding the collected volatile organic compounds by generating an electromagnetic radiation in the range of THz within a scanning window of about 100 GHz .

6. The system of any one of the preceding claims, wherein said pressure permeable membrane (104) has a thickness being at least several times the wavelength of the THz radiation.

7. A method (200) for determining one or more egg properties immediately after laying and prior to incubation, the method (200) comprising:

   collecting volatile organic compounds (208) by a pressure permeable membrane (104);
   scanning (210) said collected volatile organic compounds, which are trapped in said pressure permeable membrane (104), with electromagnetic radiation in the THz range;
   detecting (210), by a detection unit (108B), the THz radiation after having passed through the collected volatile organic compounds trapped in the pressure permeable membrane (104), wherein the detection unit (108B) is disposed at a distance from the pressure permeable membrane (104), and wherein the distance is less than a wavelength of the THz radiation;
   receiving (202) via the detection unit (108B) a response THz signal indicative of collected volatile organic compounds being scanned with electromagnetic radiation in the THz range; and
   processing (204) said response THz signal for identifying a THz signature being indicative of at least one of gender and fertility.

8. The method of claim 7, wherein said processing (204) comprises performing a pattern recognition (206) of said THz signature.

9. The method of claim 7 or 8, further comprising scanning the collected volatile organic compounds with electromagnetic radiation in the THz range within a scanning window of about 100 GHz.

10. The method of any one of claims 7 to 9, further comprising trapping collected volatile organic compounds by suction within a period of time, wherein the period of time is less than a period of time spent for transporting the egg from a tray to a conveyor.

11. The method of claim 10, wherein said period of time is less than 5 seconds.


**Patentansprüche**

1. System (100) zum Bestimmen von einer oder mehreren Eigenschaften eines Eis unmittelbar nach dem Legen und vor dem Inkubieren, wobei das System (100) Folgendes umfasst:

Zumindest eine Vakuumgreifvorrichtung (102), die eine druckdurchlässige Membran (104) trägt, wobei die Vakuumgreifvorrichtung (102) ausgelegt und betreibbar ist, um ein Ei durch Ansaugen zu halten, und wobei die druckdurchlässige Membran (104) an dem Ausbreitungspfad von flüchtigen organischen Verbindungen, die von dem Ei freigesetzt werden, angeordnet ist, wobei die druckdurchlässige Membran (104) ausgelegt und betreibbar ist, um die gesammelten flüchtigen organischen Verbindungen einzufangen;
einen THz-Strahlungssender (108A), der in einem ersten Abstand zu der druckdurchlässigen Membran (104) angeordnet ist;
eine Detektionseinheit (108B), die in einem zweiten Abstand zu der druckdurchlässigen Membran (104) angeordnet ist, wobei die Detektionseinheit ausgelegt ist, um die THz-Strahlung zu detektieren, nachdem diese die gesammelten flüchtigen organischen Verbindungen, die in der druckdurchlässigen Membran (104) eingefangen sind, passiert hat; und
eine Steuereinheit (106), die ausgelegt und betreibbar ist, um über die Detektionseinheit (108B) ein THz-Antwortsignal, das die gesammelten flüchtigen organischen Verbindungen, die mit einer elektromagnetischen Strahlung im THz-Bereich abgetastet werden, anzeigt, zu empfangen und das THz-Antwortsignal zu verarbeiten, um eine Signatur zu identifizieren, die zumindest eine Eigenschaft des Eis anzeigt, um dadurch Informationsdaten zu erzeugen, die zumindest eine Eigenschaft des Eis anzeigen; **dadurch gekennzeichnet, dass** der zweite Abstand geringer ist als eine Wellenlänge der THz-Strahlung.

2. System nach Anspruch 1, wobei die Steuereinheit (106) ausgelegt und betreibbar ist, um eine Mustererkennung der Signatur durchzuführen.

3. System nach Anspruch 1 oder 2, wobei die druckdurchlässige Membran (104) ausgelegt ist, um über einen Zeitraum hinweg betrieben zu werden, um die gesammelten flüchtigen organischen Verbindungen einzufangen, wobei der Zeitraum geringer ist als ein Zeitraum, der für den Transport des Eis von einer Ablage zu einem Förderband erforderlich ist.

4. System nach Anspruch 3, wobei der Zeitraum weniger als 5 Sekunden beträgt.

5. System nach einem der vorangegangenen Ansprüche, das ferner eine Spektroskopieanordnung (108) umfasst, die eine Strahlungssendereinheit umfasst, die ausgelegt und betreibbar ist, um die druckdurchlässige Membran (104), die die gesammelten flüchtigen volatilen organischen Verbindungen hält, abzutasten, indem sie eine elektromagnetische Strahlung im THz-Bereich innerhalb eines Abtastfensters von etwa 100 GHz erzeugt.

6. System nach einem der vorangegangenen Ansprüche, wobei die druckdurchlässige Membran (104) eine Dicke aufweist, die zumindest ein Mehrfaches der Wellenlänge der THz-Strahlung ausmacht.

7. Verfahren (200) zum Bestimmen von einer oder mehreren Eigenschaften eines Eis unmittelbar nach dem Legen und vor dem Inkubieren, wobei das Verfahren (200) Folgendes umfasst:

Sammeln volatiler organischer Verbindungen (208) durch eine druckdurchlässige Membran (104);
Abtasten (210) der gesammelten flüchtigen organischen Verbindungen, die in der druckdurchlässigen Membran (104) eingefangen sind, mit elektromagnetischer Strahlung im THz-Bereich;
Detektieren (210) der THz-Strahlung, nachdem diese die gesammelten flüchtigen organischen Verbindungen, die in der druckdurchlässigen Membran (104) eingefangen sind, passiert hat, durch eine Detektionseinheit (108B);
wobei die Detektionseinheit (108B) in einem Abstand zu der druckdurchlässigen Membran (104) angeordnet ist und wobei der Abstand geringer ist als eine Wellenlänge der THz-Strahlung;

Empfangen (202) eines THz-Antwortsignals, das die gesammelten flüchtigen organischen Verbindungen, die mit einer elektromagnetischen Strahlung im THz-Bereich abgetastet werden, anzeigt, über die Detektionseinheit (108B); und

Verarbeiten (204) des THz-Antwortsignals, um eine THz-Signatur zu identifizieren, die zumindest eines aus Geschlecht und Fruchtbarkeit anzeigt.

8. Verfahren nach Anspruch 7, wobei das Verarbeiten (204) das Durchführen einer Mustererkennung (206) der THz-Signatur umfasst.

9. Verfahren nach Anspruch 7 oder 8, ferner umfassend das Abtasten der gesammelten flüchtigen organischen Verbindungen mit elektromagnetischer Strahlung im THz-Bereich innerhalb eines Abtastfensters von etwa 100 GHz.

10. Verfahren nach einem der Ansprüche 7 bis 9, ferner umfassend das Einfangen der gesammelten flüchtigen organischen Verbindungen durch Ansaugen innerhalb eines Zeitraums, wobei der Zeitraum geringer ist als ein Zeitraum, der für den Transport des Eis von einer Ablage zu einem Förderband erforderlich ist.

11. Verfahren nach Anspruch 10, wobei der Zeitraum weniger als 5 Sekunden beträgt.

**Revendications**

1. Système (100) pour déterminer une ou plusieurs propriétés d'oeuf immédiatement après la ponte et avant l'incubation, le système (100) comprenant :

au moins un préhenseur à vide (102) portant une membrane perméable à la pression (104), dans lequel ledit préhenseur à vide (102) est configuré et peut fonctionner pour maintenir un œuf par aspiration et dans lequel ladite membrane perméable à la pression (104) est située au niveau du trajet de propagation de composés organiques volatils libérés par l'œuf, ladite membrane perméable à la pression (104) étant configurée et pouvant fonctionner pour piéger les composés organiques volatils collectés ;
un émetteur de rayonnement THz (108A) agencé à une première distance de la membrane perméable à la pression (104) ;
une unité de détection (108B) située à une seconde distance de la membrane perméable à la pression (104), l'unité de détection étant configurée pour détecter le rayonnement THz après traversée des composés organiques volatils collectés piégés dans la membrane perméable à la pression (104) ; et
une unité de commande (106) configurée et pouvant fonctionner pour recevoir par l'intermédiaire de l'unité de détection (108B) un signal de réponse THz indiquant que les composés organiques volatils collectés sont balayés avec un rayonnement électromagnétique dans la plage THz et traiter ledit signal de réponse THz afin d'identifier une signature indiquant au moins une propriété d'œuf pour générer ainsi des données d'information indiquant au moins une propriété d'oeuf ;
**caractérisé en ce que** la seconde distance est inférieure à une longueur d'onde du rayonnement THz.

2. Système selon la revendication 1, dans lequel ladite unité de commande (106) est configurée et peut fonctionner pour effectuer une reconnaissance de motif de ladite signature.

3. Système selon la revendication 1 ou la revendication 2, dans lequel ladite membrane perméable à la pression (104) est configurée pour fonctionner pendant une période de temps pour piéger les composés organiques volatils collectés, dans lequel la période de temps est inférieure à une période de temps passée pour transporter l'œuf d'un plateau à un convoyeur.

4. Système selon la revendication 3, dans lequel ladite période de temps est inférieure à 5 secondes.

5. Système selon l'une quelconque des revendications précédentes, comprenant en outre un ensemble spectroscopique (108) incluant une unité d'émission de rayonnement configurée et pouvant fonctionner pour balayer ladite membrane perméable à la pression (104) maintenant les composés organiques volatils collectés en générant un rayonnement électromagnétique dans la plage THz à l'intérieur d'une fenêtre de balayage d'environ 100 GHz.

6. Système selon l'une quelconque des revendications précédentes, dans lequel ladite membrane perméable à la pression (104) présente une épaisseur égale à au moins plusieurs fois la longueur d'onde du rayonnement.

7. Procédé (200) pour déterminer une ou plusieurs propriétés d'oeuf immédiatement après la ponte et avant l'incubation, le procédé (200) comprenant les étapes consistant à :

collecter des composés organiques volatils (208) par l'intermédiaire d'une membrane perméable à la pression (104) ;

balayer (210) lesdits composés organiques volatils collectés, qui sont piégés dans ladite membrane perméable à la pression (104), avec un rayonnement électromagnétique dans la plage de THz ;

détecter (210), par l'intermédiaire d'une unité de détection (108B), le rayonnement THz après traversées des composés organiques volatils collectés piégés dans la membrane perméable à la pression (104), dans lequel l'unité de détection (108B) est disposée à une distance de la membrane perméable à la pression (104), et dans lequel la distance est inférieure à une longueur d'onde du rayonnement THz ;

recevoir (202) via l'unité de détection (108B) un signal THz de réponse indiquant des composés organiques volatils collectés en cours de balayage avec un rayonnement électromagnétique dans la plage THz ; et

traiter (204) ledit signal THz de réponse identifiant une signature THz indiquant au moins l'un du genre et de la fertilité.

8. Procédé selon la revendication 7, dans lequel ledit traitement (204) comprend l'exécution d'une reconnaissance de forme (206) de ladite signature THz.

9. Procédé selon la revendication 7 ou 8, comprenant en outre l'étape consistant à balayer les composés organiques volatils recueillis avec un rayonnement électromagnétique dans la plage THz à l'intérieur d'une fenêtre de balayage d'environ 100 GHz.

10. Procédé selon l'une quelconque des revendications 7 à 9, comprenant en outre le piégeage de composés organiques volatils collectés par aspiration dans une période de temps, dans lequel la période de temps est inférieure à une période de temps passée à transporter l'œuf d'un plateau à un convoyeur.

11. Procédé selon la revendication 10, dans lequel ladite période de temps est inférieure à 5 secondes.

100

Spectroscopic assembly 108

| THz radiation transmitter unit 108A | Detection unit 108B |

Vacuum gripper 102

Pressure dischargeable capacitor 104

VOCs

Egg

Control unit 106

| Data input 106A | Memory 106C |

Processing utility 106D

Data output 106B

Egg properties

Fig. 1

102

Fig. 2A

104

Fig. 2B

200

214 — Obtaining reference spectrum

208 — Trapping collect VOCs

210 — Performing THz spectroscopy

202 — Receiving data indicative of VOCs ---- 212 — Recording THz signature in a database

204 — Processing data and identifying signature

Pattern recognition 206

Gender and/or fertility

Fig. 3A

300

Processing data and identifying signature

310 — Preprocessing

312 — Feature extraction

314 — Pattern classification

Fig. 3B

## Male

Fig. 4A

## Female

Fig. 4B

## Fresh permeable capacitor

Fig. 4C

Fig. 5A

Female average spectral responce

Intensity

Measured points

Fig. 5B

Male average spectral responce

Intensity

Measured points

EP 3 658 893 B1

Fig. 6

EP 3 658 893 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018023105 A1
- US 4955728 A
- US 4671652 A
- US 20160050891 A1
- US 20040107912 A1
- US 20090091742 A1
- WO 2014086335 A1
- WO 2015145435 A1
- US 9279723 B2

**Non-patent literature cited in the description**

- Gender Identification of Chicks Prior to Hatch. *50™ Annual National Breeders Roundtable St. Louis, Missouri,* 03 May 2001
- **WEBSTER B ; HAYES W ; PIKE TW.** Avian Egg Odour Encodes Information on Embryo Sex, Fertility and Development. *PLoS ONE,* 2015, vol. 10 (1), e0116345
- *Opt Express.,* 09 February 2015, vol. 23 (3), 2048-57